# EUROPEAN PATENT APPLICATION

(11) **EP 2 870 957 A1**
(43) Date of publication of application: **13.05.2015**
(21) Application number: 13191926.8
(22) Date of filing: 07.11.2013
(51) Int. Cl.: A61K 8/44, A61K 8/46, A61Q 5/02, A61Q 19/10, A61K 8/42

(54) **Optically clear isethionate aqueous concentrate for cosmetic use**

(71) Applicant: OTC GmbH, 46047 Oberhausen (DE)
(72) Inventor: Dahms, Gerd Herbert, 47138 Duisburg (DE)
(74) Representative: Michalski Hüttermann & Partner Patentanwälte mbB

(57) **Abstract**

The present invention relates to optically clear, aqueous isethionate concentrates comprising sparingly water soluble acyl-isethionate above the water solubility-threshold, characterised in that the concentrate comprises sparingly water soluble acyl-isethionates, glucamides, acyl-amino acids in an overall amount of ≥ 50 weight-% and ≤ 80 weight-%, at least 5 weight-% water, and the concentrate turbidity at 20°C according to DIN EN ISO 7027 is ≥ 0,01 NTU and ≤ 150 NTU.

## Description

The present invention relates to optically clear, aqueous isethionate concentrates comprising sparingly water soluble acyl-isethionate above the water solubility-threshold, characterised in that the concentrate comprises sparingly water soluble acyl-isethionates, glucamides, acyl-amino acids in an overall amount of ≥ 50 weight-% and ≤ 80 weight-%, at least 5 weight-% water, and the concentrate turbidity at 20°C according to DIN EN ISO 7027 is ≥ 0,01 NTU and ≤ 150 NTU.

Functional compounds in dermatological formulations may suitably be used if it is possible to achieve the right concentration of the active species at the right spot. Consequently, also situations have to be kept in mind, where the active species appears in a sufficient concentration at the wrong target. This unwanted possibility is usually proportional to the general performance of the compound itself and, keeping in mind a dermatological background, directly linked to adverse effects. Nevertheless, in some cases it is also possible to indentify compounds, which exhibit, due to the chemistry of the molecules itself, at the same time a high performance and excellent safety profile.

In the field of cleaning applications it is known for a long time that isethionates in general do exhibit very good cleaning and foaming performance and, compared to the standard anionic tensides, superior skin compatibility at reasonable costs. Unfortunately, due to their poor solubility in water, the formulations have long time been limited to solid cleansing products like syndet or syndet/soap bars. Here the solid composition prevents re-crystallisation of the isethionate, but only non-transparent formulations were available.

In order to overcome the isethionate obstacle of poor water solubility several approaches has been proposed in the literature and in patent documents. Sun et al. for instance describes three different ways of formulating poorly soluble sodium cocoyl isethionates into stable liquid products (Sun J.Z. et al., J. Cosmet. Sci., 54, 559-568, Nov/Dec 2003). Suitable strategies may include 1) incorporation of poorly soluble isethionates into secondary surfactant micelles, 2) exchange of the sodium counter-ions with ammonium-ions and 3) emulsification of the isethionates and subsequent change of micelles into emulsified oil drops.

The secondary surfactant approach including micelles is for instance realised in EP0964674 A2, disclosing a concentrated mixture of three or more surfactants dissolved or dispersed in a stable form in water comprising an acyl isethionate, an imidazoline amphoteric surfactant, and at least one additional anionic surfactant.

Another secondary surfactant composition is disclosed in US 2013/0189212 A1. This document describes a system comprising (a) 1 to 20 weight-% of at least one fatty acyl isethionate compound; (b) 0.1 to 10 weight-% of at least one acyl glycinate compound; (c) 0.1 to 20 weight-% of at least one alkyl betaine compound; and (d) 60 to 98.8 weight-% of water wherein the weight ratio of fatty acyl isethionate to acyl glycinate and alkyl betaine is in the range of 1:0.1 to 1:1 and the composition is clear, concentrated and flowable at and below 25 °C.

Furthermore, EP 2 033 624 A2 discloses an aqueous concentrate (I) comprising: an isethionate compound (at 0.1-8 wt.%); a taurate compound (at 0.1-8 wt.%); and an alkyl betaine compound (at 0.1-40 wt.%), where the sum of all the surfactants is greater than 20 wt.%. In addition, special structures of the single tensides are disclosed, which should enable a stable isethionate formulation.

Nevertheless, although some approaches and detailed recipes are given in the literature in order to formulate stable isethionate compositions it is still difficult to find a suitable cost effective solution for liquid formulation systems, which are able to achieve dermatological acceptable, high foaming and optical clear aqueous products.

### SUMMARY OF THE INVENTION

Therefore, it is the task of the present invention to provide a freely flowable and essentially optically clear isethionate concentrate comprising sparingly water soluble isethionates, which is very tolerant to incorporation of other ingredients, can additionally be thickened by addition of sodium chloride and which is easily dilutable in water.

### DETAILED DESCRIPTION OF THE PREFERRED EMBODIMENTS

This object is achieved according to the invention by an optically clear, aqueous isethionate concentrate comprising sparingly water soluble acyl-isethionate above the water solubility-threshold, characterised in that the concentrate comprises sparingly water soluble acyl-isethionates, glucamides, acyl-amino acids in an overall amount of ≥ 50 weight-% and ≤ 80 weight-%, at least 5 weight-% water, and the concentrate turbidity at 20°C according to DIN EN ISO 7027 is ≥ 0,01 NTU and ≤ 150 NTU. Surprisingly it has been found that the inventive concentrate exhibits several advantages with respect to product appearance, processing and storage behaviour and dermatological efficacy. Although within the concentrate isethionates are incorporated above their solubility threshold, which usually leads to the formation of macroscopic precipitates upon storage, the resulting concentrate is optically clear or only slightly opaque. This is an indication that the isethionates are solubilised by the ingredients, namely other co-surfactants and only small surfactant structures are formed, resulting in a favourable, clear product appearance. The concentrate is storage stable at room temperature and even at lower temperatures, without precipitation of the isethionates. The processing of the inventive concentrate is easy and solubilisation of the isethionates can be achieved by only low shear force mixing in the cold (below 75°C). The concentrate exhibits even at such high active contents a low viscosity, i.e. the concentrate is flowable, which eases handling. Upon introduction of air the concentrate shows a good lather. The resulting foam is smooth and stable and very tolerant with respect to addition of other substances which usually lead to a drastic decrease in foaming behaviour. The overall formulation is very effective with respect to cleaning behaviour and, in addition, is dermatological safe.

Without being bound by the theory the solubilisation of the isethionates is achieved by the formation of lamellar co-surfactant structures, which are able to incorporate dissolved isethionate molecules, thus shifting the solubility equilibrium between the solid and dissolved isethionates completely to the right. Therefore, it is possible to increase the water solubility of the sparingly water soluble isethionates up to 50 weight-% in aqueous solution. Due to the fact that the isethionates are integrated into lamellar co-surfactant structures instead, according to state of the art, micellar co-surfactant structures, the re-crystallization of the isethionates during storage and/or upon cooling is effectively prevented, resulting in a storage stable system. This finding is valid for a broad range of compositions where also additional ingredients like actives, preservatives, perfumes, pigments, oils etc. can be integrated. Furthermore, it has been found that besides the dissolution properties and the storage stability the inventive lamellar co-surfactant/isethionate structures yield excellent foaming behaviour. High foam volumes are achievable just by manual friction and the resulting foam exhibits excellent stability.

The lamellar co-surfactant structures are very suitable to integrate sparingly water soluble isethionates. In a preferred embodiment of the invention also very high isethionates concentrations can be dissolved. Such very high isethionates concentrations may be in between 25 weight-% and 50 weight-% and even higher isethionate concentrations in between 35 weight-% up to 50 weight-%. Even such high sparingly soluble isethionates concentrations can be dissolved by the inventive composition and the composition remains optically clear.

The compositions according to the invention are optically clear, i.e. the composition is either optically clear or only slightly opaque, i.e. only a slight scattering, possibly due to tyndall-scattering, might be visible. This is in contrast to state of the art isethionate co-surfactant systems, which are, due to un-dissolved isethionates, usually non-transparent solids. Favorably, it is possible to achieve optically clear or only slightly opaque aqueous isethionate solution by using the inventive co-surfactant composition. Without being bound by the theory this is achievable, because the isethionates are readily dissolved by the uptake into the lamellar co-surfactant structures and the lamellar aggregate size is small enough to prevent light scattering. Higher NTU values are not within the scope of the invention, because then the overall composition becomes too translucent, which might affect consumer acceptance. The NTU-values of the composition may be assessed according to known methods in the art, for instance according to DIN EN ISO 7027. It may also be within the scope of the invention to provide optically clear compositions comprising NTU-values smaller or equal to 125 NTU, or even comprising NTU-values smaller or equal to 100 NTU.

Sparingly soluble acyl-isethionate isethionates according to the invention are acyl-isethionates, which exhibit solubility in demineralised water of less than 3 g/L at 20°C. The acyl-isethionates comprise a structure according to the following formula 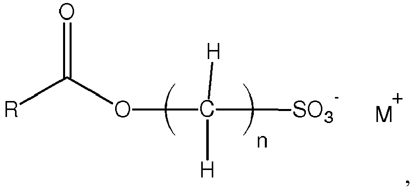 wherein R is an alkyl-group of 8 to 22 carbon atoms, M⁺ is a monovalent-cation and n is an integer ranging from 1-4. As a function of the acyl-chain length, n and the counter-cation the isethionates do show a distinct solubility in water, wherein usually the sodium isethionates exhibit very low water solubility, if formulated alone. One classical example for such sparingly water soluble isethionates is sodium cocoyl-isethionate, comprising a coco-group and n = 2. Within a preferred embodiment of the invention isethionates may be used, wherein n is equal to 2. Additionally, it is also within the scope of the invention that isethionate mixtures of different carbon chain length can be used. The solubility of the isethionates in water as a function of the carbon chain length and the counter-cation are either tabulated or can be assessed using standard physical methods. One possible method is the determination of the solution conductivity as a function of the isethionate concentration.

According to the invention glucamides are used as co-surfactants, which are able to form lamellar systems. Glucamides belong to the class of non-ionic surfactant and comprise the following formula 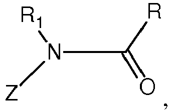 wherein R₁ can be selected from the group comprising H, C1-C4 alkyl, 2-hydroxy ethyl, 2-hydroxy propyl, or a mixture thereof. Preferably R₁ comprises C₁-C₄ alkyl, more preferably C₁ or C₂ alkyl, most preferably C₁ alkyl (i.e., methyl). R can be selected from the group comprising C₅-C₃₁ hydrocarbyl, preferably straight chain C₇-C₁₉ alkyl or alkenyl, more preferably straight chain C₉-C₁₇ alkyl or alkenyl, most preferably straight chain C₁₁-C₁₇ alkyl or alkenyl, or mixtures thereof. R-CO-N< can be, for example, cocamide, stearamide, oleamide, lauramide, myristamide, capricamide, palmitamide, tallowamide, etc.. Z is a polyhydroxyhydrocarbyl having a linear hydrocarbyl chain with at least 3 hydroxyls directly connected to the chain or an alkoxylated derivative (preferably ethoxylated or propoxylated) thereof. Z preferably will be derived from a reducing sugar in a reductive amination reaction; more preferably Z is a glycityl. Suitable reducing sugars include glucose, fructose, maltose, lactose, galactose, mannose, and xylose. As raw materials, high dextrose corn syrup, high fructose corn syrup, and high maltose corn syrup can be utilized as well as the individual sugars listed above. These corn syrups may yield a mix of sugar components for Z. Z preferably will be selected from the group consisting of -CH₂-(CHOH)ₘ-CH₂OH, -CH(CH₂OH)-(CHOH)ₘ₋₁-CH₂OH, - CH₂-(CHOH)₂- (CHOR')(CHOH)-CH₂OH, where m is an integer from 3 to 5 and R' is H or a cyclic or aliphatic monosaccharide, and alkoxylated derivatives thereof. Most preferred are glycityls wherein m is 4, particularly -CH₂-(CHOH)₄-CH₂OH. Z can be for instance 1-deoxyglucityl, 2-deoxyfructityl, 1-deoxymaltityl, 1-deoxylactityl, 1-deoxygalactityl, 1-deoxymannityl, 1-deoxymaltotriotityl, etc..

Acyl-aminoacids according to the invention may either be acylated aminoacids or acylated peptides usually used in detergent formulations. Acylated peptides which may be used in the present invention are those which may be obtained by hydrolyzing a naturally occurring protein to produce a peptide having an average molecular weight of 200 to 8,000, followed by acylating the peptide with an acylating agent having 6 to 24 carbon atoms. Salts of the acylated peptides include alkali metal salts, hydroxyalkyl-substituted ammonium salts and ammonium salts. The hydroxyalkyl-substituted ammonium salt may preferably have 1 to 3 carbon atoms in the hydroxyalkyl group. These acylated peptides and salts thereof may be used independently or in combination of two or more.
Illustrative examples of these compounds include N-cocoyl peptides, N-myristyl peptides, N-oleyl peptides, N-undecylyl peptides, and their alkali metal salts, hydroxyalkyl-substituted ammonium salts, and the like. In particular, N-cocoyl peptides, their alkali metal salts and hydroxyalkyl-substituted ammonium salts, and N-oleyl peptides, their alkali metal salts and hydroxyalkylsubstituted ammonium salts are preferably used in the present invention. The hydroxyalkyl substituted ammonium salts may preferably have 1 to 3 carbon atoms in the hydroxyalkyl group.

Acyl groups in the N-acyl amino acids and salts thereof which may be used in the present invention have 6 to 24 carbon atoms; for example, lauroyl, myristoyl, palmitoyl, or the like is included. The amino acids include glutamic acid, glycine, beta-alanine and the like. The salts include alkali metal salts, hydroxyalkyl-substituted ammonium salts and ammonium salts. The hydroxyalkyl substituted ammonium salts may preferably have 1 to 3 carbon atoms in the hydroxyalkyl group. N-acyl-N-alkyl amino acids are also included in the term "N-acyl amino acids" used herein. The alkyl groups in the N-acyl-N-alkyl amino acids may preferably have 1 to 3 carbon atoms and include methyl, ethyl, propyl, isopropyl and the like. These N-acyl amino acids and salts thereof may be used independently or in combination of two or more.

Preferred N-acyl amino acids and salts thereof may include N-acyl amino acids such as N-laurolylglutamic acid, N-myristoylglutamic acid, N-palmitoylaglutamic acid, N-myristoyl-beta-alamine, N-palmitoyl-beta-alanine and the like; N-acyl N-alkyl amino acids such as N-lauroyl-N-ethylglycine, N-lauroyl-N-isopropylglycine, N-lauroylsarcosine, N-myristoylsarcosine, N-palmitoylsarcosine, N-lauroyl-N-methyl-beta-alanine and the like; as well as their alkali metal salts, hydroxyalkyl-substituted ammonium salts and the likes.

In an embodiment of the invention the optically clear isethionate aqueous concentrate comprise a viscosity at a combined active concentration of the isethionate, glucamides and acyl-aminoacids of larger than 50 weight-% of ≥ 1000 mPa s and ≤ 100000 mPas, preferably > 2000 mPas and ≤ 75000 mPas and additionally ≥ 5000 mPas and ≤ 50000 mPas. This special viscosity range may be helpful in providing a concentrate which may easily be foamed only by mechanical means. In addition, the lower viscosity limit may be helpful to provide a composition with sufficient body, which is not immediately flowing from the body surface. Such viscosity range is also helpful for an easy handling of the concentrate in production. The composition viscosity can be determined according to methods known to the skilled in the art, for instance using a TA-Instruments plate/plate viscosimeter with a gap of 200 µm. The viscosity is determined at 25°C at a shear rate of 10 1/s.

These and other aspects of the invention will be apparent from and elucidated with reference to the embodiments described hereinafter.

In a preferred embodiment of the invention the concentrate comprises Newtonian flow behaviour at 20 °C and shear rates ≥ 0,1 1/s and ≤ 100 1/s, measured in a plate/plate configuration and a gap of 200 µm. Surprisingly it has been found that the concentrate exhibits a viscosity, which is extremely low for such kind of systems and shear rare independent, i.e. constant, in the above mentioned shear rate range. Such Newtonian flow behaviour eases the processing of the concentrate. Pumps and pipes can advantageously be adapted to such flow behaviour.

In an additional aspect of the invention the concentrate turbidity at 5°C according to DIN EN ISO 7027 is ≥ 0,01 NTU and ≤ 90 NTU. In addition to a clear appearance of the concentrate at roughly room temperature (20°C) it has surprisingly been found that even in the cold the concentrate remains clear. Without being bound by the theory this might be achieved by a stable incorporation of the sparingly soluble isethionates in the inventive co-surfactant system, also depressing the crystallization tendency of the sparingly water soluble isethionates at lower temperatures. This behavior advantageously increases the storage stability of the overall system.

Furthermore, the concentrate may comprise sparingly water soluble acyl-isethionates in an amount of ≥ 3,0 weight-% and ≤ 50 weight-%, at least one Glucamide in an amount of ≥ 1,0 weight-% and ≤ 50 weight-%, at least one acyl-amino acid in an amount ≥ 0,1 weight-% and ≤ 20 weight-% and the overall concentration of these compounds in the concentrate is ≥ 50 weight-% and ≤ 80 weight-%. Such concentration range of the sparingly soluble isethionates and the co-surfactants has been rendered useful to achieve a good combination of application and processing properties. Here especially it has to be mentioned that the foam behaviour is excellent, the optical appearance is clear to only slightly opaque and the viscosity of concentrate can easily be handled in processing and application. Within the above given surfactant concentration the sparingly water soluble acyl-isethionate concentration may also be ≥ 10,0 weight-% and ≤ 40 weight-% and preferably ≥ 15,0 weight-% and ≤ 30 weight-%. Such compositions especially yield good foam behaviour and are useful for treating sensitive skin and include good skin conditioning properties. Also within that given concentration range the glucamid concentration may suitably be ≥ 10,0 weight-% and ≥ 40 weight-% and preferably > 20,0 weight-% and ≤ 35 weight-%. Furthermore, the acyl-amino-acid concentration may suitably be ≥ 4,0 weight-% and ≤ 15 weight-% and preferably ≥ 6,0 weight-% and ≤ 12 weight-%.

Another embodiment of the invention comprise the inventive concentrate, wherein the viscosity of the concentrate measured at 20°C at a shear rate of 10 1/s is ≥ 1000 mPa s and ≤ 50000 mPa s. The viscosity is determined as given above. This viscosity range is compared to the viscosity of the standard isethionate systems very low. Therefore, the handling of the concentrate in production and application is advantageously facilitated. In a further embodiment of the invention the upper viscosity of the concentrate may be smaller or equal to ≤ 25000 mPa s, furthermore preferably ≤ 15000 mPa s.

In an additional characteristic of the invention the concentrate further comprises a non-ionic tenside selected form the group consisting of alkyl polyglucoside, polysorbate, polyethylene-glycol, polypropylene-glycol. Surprisingly it has been found that especially the above mentioned non-ionic surfactants, which do comprise only very limited foaming properties, might successfully be integrated in the inventive composition and result in high foam volumes even at low friction forces. A nice lather can be achieved used by rubbing the composition between the hands. Suitable examples of the non-ionic surfactants mentioned in the group above can be found in the CTFA International Cosmetics Ingredient Dictionary and Handbook, "Surfactants", 10th edition (2004). Non limiting examples for additional use in the inventive composition are EP alkyl-polyglucosides as mentioned in the EP0070074, polysorbates, for instance sorbitan monolaurate, sorbitan monopalmitate, sorbitan monostearate, sorbitan tristearate, sorbitan monooleate and PEGs and PPGs as known to the skilled in the art exhibiting molecular weights below 5000 Da.

Within a further object of the invention the composition comprises an oil component in an amount of ≥ 0,1 weight-% and ≤ 15 weight%. Such amounts of cosmetic oils can be incorporated into the composition without affecting the isethionate dissolution, the foaming behaviour, optical appearance and/or storage stability of the composition. The oil component may contribute to the skin compatibility of the composition and may also be helpful for cleaning purposes. Suitable oils may be selected from the group of natural, synthetic or silicon oils. Cosmetic or dermatological acceptable oils are known to the skilled in the art. Surprisingly it has additionally been found that the viscosity of the concentrate remains mainly unaffected by the addition of the oil component. Therefore, the concentrate is very tolerant to the incorporation of oils, resulting in an easy processing and application of concentrates according to the invention, also including different oils and varying oil contents.

Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, olive oil or soya oil, and the derivatives thereof.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Further, suitable synthetic oil components are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl palmitate, etc.

Silicone oils can either be volatile and/or non-volatile oils. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC1401 , DC 1403, DC 1501 and DC 1503. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl penta-phenyl trisiloxane can be advantageously comprised in the compositions of the present invention.

Surprisingly at has been found that concentrates according to the invention also including oil components remain optically clear, i.e. the concentrate turbidity at 5°C according to DIN EN ISO 7027 is ≥ 0,01 NTU and ≤ 100 NTU. The inventive isethionate/co-surfactant system is also able to include rather large amounts of non polar substances like cosmetic oils or triglycerides without turning into a very translucent system. Such behavior might be caused by a homogeneous incorporation of rather small oil-droplets into the surfactant/co-surfactant structures. Therefore even such oil-containing systems remain optically clear. This finding is especially valid in the low temperature range, where state-of the art isothionate/oil-compositions are usually very opaque.

In a preferred embodiment of the invention the composition may comprise amphiphilic molecules having a critical micelle concentration (CMC) ≥ 0,01 g/L and ≤ 1 g/L in demineralised water at 20°C. Amphiphils in the sense of the invention are organic molecules exhibiting hydrophilic (water-loving, polar) and a lipophilic (fat-loving) parts. Usually such molecules are classified as detergents, tensides or soaps. Especially the use of amphiphilic molecules comprising a low critical micelle concentration in the above given range may contribute to the dermatological acceptance and skin friendliness of the formulation. Without being bound by the theory it is assumed that the amphiphilic molecules exhibiting a low CMC are less prone to direct interaction with skin lipids. Therefore, the skin lipid barrier will not be disturbed by uptake of skin lipids into the lamellar structures and hence the protective skin lipid barrier will be kept intact. As a consequence the penetration of tensides or other allergens into deeper dermal layers is prevented. The CMC of the different tensides are tabulated in the literature or can be assessed by experimental methods known to the skilled in the art. For ionic (charged) tensides conductance can be used to evaluate the CMC. For non-ionic tensides other techniques like UV- or fluorescence measurements may be used. A further embodiment of the invention comprises a composition, wherein all surfactants and co-surfactants present in the composition comprise a CMC ≥ 0,01 g/L and ≤ 1 g/L, preferably ≥ 0,01 g/L and ≤ 0,5 g/L and most preferred ≥ 0,01 g/L and ≤ 0,25 g/L.

In another preferred aspect of the invention the composition comprises lamellar liquid crystalline aggregates. It has surprisingly been found, that especially co-surfactant systems, which form lamellar liquid crystalline aggregates or phases are suitable to incorporate high amounts of isethionates and result in storage stable compositions. Furthermore, it is also highly likely that the lamellar liquid crystalline structures of the isethionates/co-surfactant mixtures contribute to the good skin compatibility of the composition. The lamellar structures of the composition may either be in the form of two-dimensional layers or comprise spherical geometry. Suitable spherical geometries include uni- or multi-lamellar vesicles. These lamellar structures in contrast to standard micelle tenside systems, which comprise just single tenside layer structures. Lamellar tenside aggregates may be detected in the composition using freeze-fracture techniques, followed by visual inspection. Alternatively also electron or neutron scattering technique may be used in order to distinguish lamellar from micellar structures.

In an additional characteristic of the invention the composition comprises lamellar liquid crystalline aggregates ≥ 30 Å and ≤ 10 000 Å. The composition according to the invention may comprise lamellar liquid crystalline aggregates in the form of vesicles or layers. It has been found that these aggregates do form in a size range large enough to prevent a direct penetration of the lamellar aggregates through the skin pores. This might contribute to the good dermatological profile and foam behaviour of the composition. In addition, the formed aggregates are small enough in order to yield optically clear compositions. Therefore, this size range is especially suited to provide skin friendly compositions which do exhibit superior optical properties. This in contrast to the state of the art aqueous isethionate compositions, which usually are very opaque or do comprise micelles in solution, which can penetrate through skin pores and interact directly with the skin lipids. The aggregate sizes might be assessed by X-ray or neutron scattering techniques known to the skilled in the art. Further optical methods for size determination include polarizing light microscopy or freeze fracture technique.

In a further embodiment of the invention the glucamides comprise carbon chain length ≥ C8 and ≤ C20. Such carbon chain length of the glucamides may especially be suited to provide fast isethionate dissolution kinetics and a fast and stable formation of lamellar aggregates. Without being bound by the theory it is assumed that the packing parameter of such glucamides is favouring the formation of lamellar instead of the micellar structures due to their carbon chain to head group volume ratio.

It is also within the scope of the invention to provide a composition, which is essentially free of fatty acids. Essentially free in the meaning of the invention is a fatty acid concentration of ≤ 3% by weight, preferably ≤ 2% by weight. Free fatty acids are known co-surfactant compounds, which are able to induce dissolution of isethionates in aqueous solutions. Unfortunately, usually the free fatty acids do only form micelles in solution and lead to translucent compositions. In addition, due to their structures and head group/carbon chain ratio they are able to strongly interact with the skin lipids, which might result in an unfavourable change of the protective skin lipid barrier composition of the skin.

In an additional characteristic of the invention the composition may essentially be free of sulphate-ions. This means that in the composition are neither free sulphate ions in solution nor sulfate-ions attached to any other, especially organic molecule, present. Nowadays especially anionic tensides are used based on sulphates. The most prominent of this substance class is the sodium laureth sulfate (SLS). These tensides do show good surfactant properties, viscosity and foaming behaviour, but are able to disturb the skin barrier for instance by a wash-out of the skin lipids. Therefore, in the case of for instance cleaning sensitive skin, which already exhibits a damaged skin barrier, such kind of tensides might further weaken the skin lipid barrier. Furthermore, sulphate containing compositions may adversely affect hair colorations, here especially red coloured hair, which might reduce consumer acceptance.

In an additional embodiment according to the invention the composition is essentially free of quaternary ammonium-cations. The solubility of the isethionates in aqueous solution also strongly depends on the chemistry and structure of the counter-ion. Changing for instance from sodium cocoyl isethionate to ammonium cocoyl isethionates the solubility is dramatically increased. Therefore, the ion-exchange effect can be used in order to actively solubilise isethionates in aqueous solutions by an in-situ ion exchange process. In addition, commercial products are nowadays available, which already provide water soluble isethionates by provision of quaternary ammonium isethionates. Unfortunately, these products are very expensive and especially lately the presence of ammonium-ions in cosmetic preparation is under debate. Therefore, skin friendly compositions are preferred, which do not comprise any quaternary ammonium ions in the composition.

In another aspect of the present invention the composition further comprises amphoteric tensides in an amount of ≥ 0,1 weight-% and ≤ 10 weight-%. Especially further amphoteric tensides might be helpful in boosting the viscosity, the foam properties and increasing the cleaning properties of the composition. Suitable surfactants include any surfactant known to those skilled in the art as suitable for incorporation into a cosmetic or dermatologic composition. Especially the betain-surfactants are preferred for this purpose. Suitable examples of surfactants may be found in the CTFA International Cosmetics Ingredient Dictionary and Handbook, "Surfactants", 10th edition (2004). Some examples of amphoteric tensides may include lauramidopropyl betaine (LAPB), cocamidopropyl betaine, cocamidopropyl hydroxysultaine (CAPHS), tallow dihydroxyethyl betaine (TDHEB), sodium lauroamphoacetate (SLAA), disodium lauroamphodiacetate (DSLADA), sodium cocoamphoacetate (SCAA), disodium cocoamphodiacetate (DSCADA), C8-10 amidopropyl betaine (C 8-10- APB), disodium capryloamphodiacetate (DSCpADA), sodium cocoamphopropionate (SCAP), Disodium cocoamphodipropionate (DSCADP).

Furthermore, it is within the scope of the invention to provide a process for production of an optically clear isethionate concentrate, comprising the steps of
a) dissolution of glucamides, acyl-aminoacids and optionally further water soluble components in water to form an anisotropic liquid crystalline lamellar phase A,
b) dispersion of the sparingly soluble acyl-isethionate and optionally further oil soluble components or oils in water forming an oil dispersion phase B,
c) combining the phases A and B under application of shear forces until a homogeneous solution is obtained, wherein the anisotropic liquid crystalline lamellar phase is maintained. Such multi-step process has been proven helpful in order to achieve optically clear or only slightly opaque isethionate aqueous solutions. The aqueous isethionate solutions are storage stable and no precipitation of the isethionates occur even at low storage temperatures. Without being bound by the theory this is achieved by the use of secondary surfactants which are able to form lamellar structures. Such structures can suitably be used in order to dissolve the sparingly soluble isethionates. Due to the fact that the secondary surfactant form lamellar instead of micellar structures, the isethionates are integrated in larger structures compared to micelles, thus leading to a better dissolution and better storage behaviour. In addition, caused by the choice of the secondary surfactant also the size of the lamellar structures can be controlled, resulting in the formation of optically clear solutions. Only at very high surfactant concentration the solution may be slightly opaque caused by the formation of larger aggregates.

Within step a) the co-surfactants are dissolved and as a result of their intrinsic packing parameter and the chosen concentration range a lamellar liquid crystalline phase is obtained. Thus, stable liquid crystalline phases a generated, either as lamellar cubic phases or non-cubic lamellar phases. In step a) ≥ 30 weight-% and ≤ 80 weight-% of the total water amount of steps a)-c) can be used, preferably ≥ 40 weight-% and ≤ 70 weight-% and additionally preferred > 50 weight-% and ≤ 70 weight-%. These water contents result in a homogenous formation of lamellar liquid crystalline phases and might additionally help to dissolve further water soluble components present in step a). Further water soluble components incorporated in step a) might exhibit water solubility at 20°C of higher than 10 g/L.

In step b) the sparingly soluble isethionates and optionally further oil soluble components are pre-dispersed in water. Such procedure is favoured, because at this stage larger isethionates agglomerates are broken and the isethionates surface is wetted. As a function of the concentration of additionally present oil soluble substances a part of the sparingly soluble isethionates might even be pre-dissolved in these substances. This might accelerate the following dissolution step.

In step c) the aqueous composition of step a) and step b) are combined under application of shear forces. Due to the inventive selection of the co-surfactants the dissolution of the sparingly isethionates can easily be achieved. The temperature at step c) can be ≥ 10 °C and ≤ 90 °C, preferably ≥ 20 °C and ≤ 85 °C and even more preferred ≥ 30 °C and ≤ 75 °C. Such temperature range may be suitable to dissolve the isethionates in reasonable processing times and might help to reduce production costs.

In a further aspect the inventive process may comprise an additional step d), wherein the concentrate of step c) is diluted by the addition of water, wherein the liquid crystalline lamellar phase is maintained. In step this additional step d) the overall concentration of the ingredients can be reduced by the addition of water. Therefore it is also possible to produce a concentrate including the sparingly soluble isethionates and dilute this concentrate for instance before a packing step. Such procedure might reduce the processing and handling costs.

Furthermore, a use of the inventive aqueous isethionate composition in a cosmetic, dermatological or pharmaceutical treatment is within the scope of the invention. As a result of the inventive surfactant/co-surfactant mixture the composition comprises only surfactants which are able to form lamellar structures and also exhibiting very low CMC. Consequently, the resulting composition is very skin friendly. Therefore, these compositions are especially suited to be used in applications, where sensitive skin has to be treated.

Optionally, the inventive aqueous isethionate composition can be used in a cosmetic rinse-off cleaning treatment. Due to their high foaming properties the inventive compositions are particularly suitable for cleaning treatments. Here especially rinse-off treatments, because the cleaning foams can easily be wiped off after application.

It is furthermore within the scope of the invention to provide a kit of parts comprising the inventive isethionate aqueous composition and at least one wipe comprising a surface roughness according to DIN EN ISO 25178 of ≥ 0,5 µm and ≤ 200 µm. Especially the combination of the inventive composition together with a wipe comprising the above mentioned surface roughness has been shown to exhibit various benefits in application. Without being bound by the theory the special surface roughness is able to provide a rich, creamy lather within a very short application time. It is assumed that this surface roughness is able to break up the surfactant lamellar structures and to easily integrate air into the composition. Therefore, the foam characteristics can be produced just by manual action of the pre-soaked wipe on the skin or by application of the inventive composition onto the pre-soaked skin and afterwards mechanical action of the wipe. Taking into account standard wipe material, suitable for cosmetic or medicinal purposes, this surface roughness ensures also that even sensitive skin surfaces are not mechanically damaged.

Other variations to be disclosed embodiments can be understood and effected by those skilled in the art in practicing the claimed invention, from a study of the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

With respect to additional advantages and features of the previously described use of the composition it is explicitly referred to the disclosure of the inventive composition. In addition, also aspects and features of the inventive composition shall be deemed applicable and disclosed to the inventive use. Furthermore, all combinations of at least two features disclosed in the claims and/or in the description are within the scope of the invention unless otherwise explicitly indicated.

### Examples:

### Preparation of the inventive composition comprising sparingly water soluble isethionates:

The composition comprises:

| | | |
|---|---|---|
| I. | Sodium Isethionate | 20 weight-% |
| II. | C12/C14 N-Methyl-Glucamide | 25 weight-% |
| III. | Sodium Cocoyl Glycinat (30 weight-%) | 25 weight-% |
| IV. | Water | 30 weight-% |

The inventive composition is achieved by:
a) Preparation of a first phase by combining the C12/C14-Glucamide and the Sodium Cocoyl Glycinat (30 weight-% solid content, remainder water) at 50°C under gentle stirring. In principle for this step the temperature may suitably be chosen in between 45°C - 60°C.
b) In a second vessel the Sodium Isethionate is dissolved in water under gentle stirring at 70 °C.
c) Phase b) is combined with phase a) by slowly pouring the Isethionate solution into phase a) under gentle stirring of the mixture. The stirring force should be adapted in a way that a laminar flow regime is achieved in the vessel.
d) Cooling of the combined phases to room temperature.

The resulting composition is optically clear and comprises a viscosity of 10 000 mPa s (25°C, plate/plate geometry, shear rate 1 1/s, 200 µm gap, TA-Instruments).

## Claims

1. Optically clear, aqueous isethionate concentrate comprising sparingly water soluble acyl-isethionate above the water solubility-threshold,
**characterised in that** the concentrate comprises sparingly water soluble acyl-isethionates, glucamides, acyl-amino acids in an overall amount of ≥ 50 weight-% and ≤ 80 weight-%, at least 5 weight-% water, and the concentrate turbidity at 20°C according to DIN EN ISO 7027 is ≥ 0,01 NTU and ≤ 150 NTU.

2. Concentrate according to claim 1, wherein the concentrate comprises Newtonian flow behaviour at 20 °C and shear rates ≥ 0,1 1/s and ≤ 100 1/s, measured in a plate/plate configuration and a gap of 200 µm.

3. Concentrate according to any of the preceding claims, wherein the concentrate turbidity at 5°C according to DIN EN ISO 7027 is ≥ 0,01 NTU and ≤ 90 NTU.

4. Concentrate according to any of the preceding claims, wherein the concentrate comprise sparingly water soluble acyl-isethionates in an amount of ≥ 3,0 weight-% and ≤ 50 weight-%, at least one Glucamide in an amount of ≥ 1,0 weight-% and ≤ 50 weight-%, at least one acyl-amino acid in an amount ≥ 0,1 weight-% and ≤ 20 weight-% and the overall concentration of these compounds in the concentrate is ≥ 50 weight-% and ≤ 80 weight-%.

5. Concentrate according to any of the preceding claims, wherein the viscosity of the concentrate measured at 20°C at a shear rate of 10 1/s is ≥ 1000 mPa s and ≤ 50000 mPa s.

6. Concentrate according to any of the preceding claims, wherein the concentrate further comprises a non-ionic tenside selected form the group consisting of alkyl polyglucoside, polysorbate, polyethylene-glycol, polypropylene-glycol.

7. Concentrate according to any of the preceding claims, wherein the concentrate is essentially free of fatty acids.

8. Concentrate according to any of the preceding claims, wherein the concentrate is essentially free of sulphate-ions.

9. Concentrate according to any of the preceding claims, wherein the concentrate further comprises an oil component in an amount of ≥ 0,1 weight-% and ≤ 15 weight-%.

10. Concentrate according to claim 9, wherein the concentrate turbidity at 5°C according to DIN EN ISO 7027 is ≥ 0,01 NTU and ≤ 100 NTU.

11. Process for production of an optically clear isethionate concentrate according to any of the preceding claims, comprising the steps of
a) dissolution of glucamides, acyl-aminoacids and optionally further water soluble components in water to form an anisotropic liquid crystalline lamellar phase A,
b) dispersion of the sparingly soluble acyl-isethionate and optionally further oil soluble components or oils in water forming an oil dispersion phase B,
c) combining the phases A and B under application of shear forces until a homogeneous solution is obtained, wherein the anisotropic liquid crystalline lamellar phase is maintained.

12. Process according to claim 11, wherein in an additional step d) the concentrate of step c) is diluted by the addition of water, wherein the liquid crystalline lamellar phase is maintained.

13. Use of an isethionate concentrate according to any of the claims 1-10 in a cosmetic, dermatological or pharmaceutical treatment.

14. Use according to claim 13, wherein the treatment is a cosmetic rinse-off cleaning treatment.

15. Kit of parts comprising the concentrate according to any of the claims 1-10 and at least one wipe comprising a surface roughness according to DIN EN ISO 25178 of ≥ 0,5 µm and ≤ 200 µm.
